# EUROPEAN PATENT APPLICATION

(11) **EP 2 289 878 A1**
(43) Date of publication of application: **02.03.2011**
(21) Application number: 10157475.4
(22) Date of filing: 31.05.2005
(51) Int. Cl.: C07D 211/22

(54) **Fexofenadine polymorphs and process for the preparation thereof**

(30) Priority: 08.06.2004 IT MI20041143
(62) Divisional of application: 05011652.4
(71) Applicant: Dipharma S.p.A., 33036 Mereto di Tomba (Udine) (IT)
(72) Inventor: Castaldi, Graziano, 28072, BRIONA (NO) (IT); Barreca, Giuseppe, 23874, MONTEVECCHIA (LC) (IT); Allegrini, Pietro, 20097, S. DONATO MILANESE (MI) (IT); Ventimiglia, Gianpiero, 20092, CINISELLO BALSAMO (MI) (IT)
(74) Representative: Minoja, Fabrizio

(57) **Abstract**

The invention provides a novel crystalline form of fexofenadine hydrochloride monosolvate with acetonitrile, a process for its preparation and its conversion into the known form A, and its use in therapy.

## Description

### FIELD OF THE INVENTION

The present invention relates to novel crystalline forms of fexofenadine hydrochloride, in particular a hydrated form and a solvate with acetonitrile, their use in therapy and a process for their preparation. The invention further relates to a process for the preparation of the known polymorphic form A of anhydrous fexofenadine hydrochloride.

### TECHNOLOGICAL BACKGROUND

Fexofenadine hydrochloride (4-[4-[4-(hydroxydiphenylmethyl)-1-piperidinyl]-1-hydroxybutyl]-α,α-dimethylbenzeneacetic acid hydrochloride) 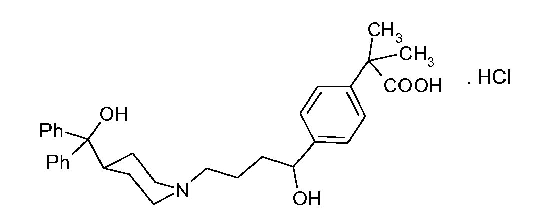 is an antihistaminic, antiallergic and bronchodilator medicament, marketed in the USA under the tradename Allegra^{®}.

WO 02/066429 discloses anhydrous fexofenadine hydrochloride polymorphic form A and a process for the preparation thereof.

US 2003/0021849 discloses other crystalline forms of fexofenadine hydrochloride, such as solvates with methyl butyl ether (MTBE) and cyclohexane, referred to as MBTE form IX and cyclohexane form IX, as well as solvates with ethyl acetate, referred to as forms XIV and XV.

WO 95/31437 discloses the preparation of polymorphs, or pseudopolymorphs and hydrates of fexofenadine hydrochloride (form II and IV) and their conversion to anhydrous polymorphic forms (form I and II) by azeotropical distillation or recrystallization to "minimize the water content".

Different forms of biologically active compounds, such as polymorphs, are known to have different bioavailability, release time and solubility, which allow, for example, dose reduction or prolonged administration intervals. Moreover, the different physical properties that are often associated to different physical forms of medicaments can be advantageously exploited in the manufacture of pharmaceutical formulations.

There is therefore the need to provide novel polymorphic forms of biologically active compounds with advantageous properties.

### SUMMARY OF THE INVENTION

In a first aspect the invention provides a novel approximately monohydrate crystalline form of fexofenadine hydrochloride, hereinafter referred to as form B, and a method for its preparation.

In a second aspect the invention provides a novel crystalline form of fexofenadine hydrochloride, approximately monosolvate with acetonitrile, hereinafter referred to as form C, and a method for its preparation.

In a further aspect, the invention provides the use of fexofenadine hydrochloride form C for the preparation of the known form A.

Furthermore, the invention provides a pharmaceutical composition containing fexofenadine hydrochloride form B and/or C as active ingredient in admixture with an excipient and/or carrier, and its use in therapy.

### DESCRIPTION OF THE FIGURES

The novel crystalline forms B and C and the known form A were characterized by XRPD (X-ray powder diffraction) technique. The X-ray diffraction spectra (XRPD) were registered with an APD 2000 θ/θ automatic diffractometer for powders and liquids (Ital-Structures), under the following operative conditions: radiation CuKα (λ = 1.5418 Å), scanning with a 0.03°C angular step for 1 sec. The DSC (Differential Scanning Calorimetry) spectra were recorded with a DSC 822e calorimeter (Mettler-Toledo), in a temperature range of 30-250°C, with a scanning rate of 10°C/minute. The ¹H-NMR spectra were recorded with a Varian Mercury 300 spectrometer, using DMSO-d₆ as the solvent.
Figure 1. XRPD spectrum (X-ray powder diffraction) of fexofenadine hydrochloride form B.
Figure 2. DSC spectrum of fexofenadine hydrochloride form B.
Figure 3. XRPD spectrum of fexofenadine hydrochloride form C.
Figure 4. ¹H-NMR spectrum of fexofenadine hydrochloride form C.

### DETAILED DISCLOSURE OF THE INVENTION

According to the present invention, "approximately monohydrate" means that the crystalline solid has a water content of about 0.8-1.2 mols per mol of fexofenadine hydrochloride, preferably from about 0.9 to 1.1 mols. "Approximately monosolvate" means that the crystalline solid has an acetonitrile content of about 0.8-1.2 mols of solvent per mol fexofenadine hydrochloride, preferably from 0.9 to 1.1 mols.

As used in the following, the term precipitation (or "precipitate") has the same meaning as crystallization (or "crystal"), and indicates the obtainment of a solid form.

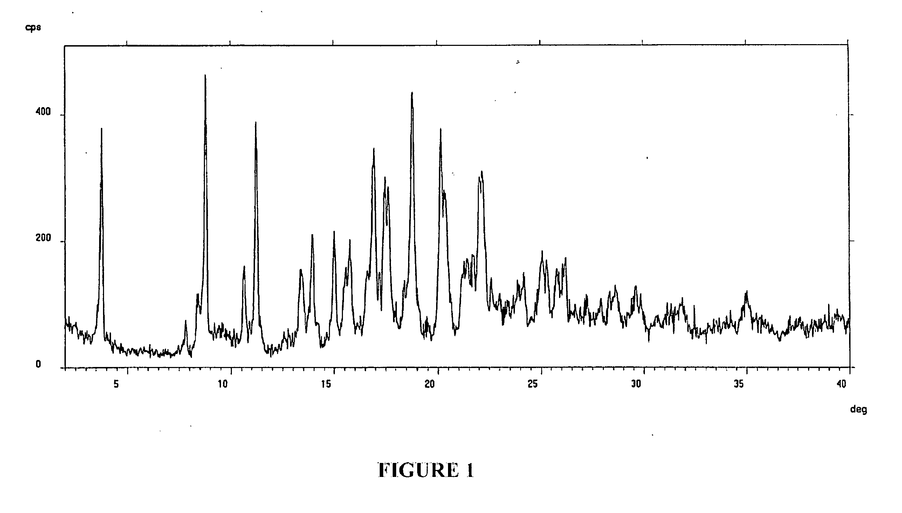

The present invention relates to a novel approximately monohydrate, crystalline form of fexofenadine hydrochloride, hereinafter defined as form B, having an XRPD spectrum substantially as illustrated in Figure 1, wherein the more intense diffraction peaks fall at 23.6; 10.1; 7.9; 5.2; 4.7 and 4.4 in 2θ, and a DSC spectrum having three endotermic peaks at about 80.27; 109.27 and 149.14°C; said form B being prepared through a process comprising the following steps:
- preparation of a solution of fexofenadine hydrochloride in a methanol/water mixture;
- cooling of the mixture at a temperature lower than -5°C; and
- separation of the precipitate.

Fexofenadine hydrochloride can be obtained by reduction of 4-[4-[4-(hydroxydiphenylmethyl)-1-piperidinyl]-1-oxobutyl]-α,α-dimethylbenzeneacetic acid, or an alkyl ester thereof, with a suitable reducing agent, optionally followed by hydrolysis, as known in the art.

The methanol/water ratio in the mixture containing the solution of fexofenadine hydrochloride is for example between 0.8 and 1.25 v/v, preferably from 0.9 and 1.1 v/v, in particular around 1 v/v.

The mixture is usually cooled slowly, preferably at a temperature ranging from -10 to -25°C, more preferably from -12 to -20°C.

The precipitate is filtered off or decanted, preferably filtered off and, after washing with a water-methanol mixture in a volume ratio of 0.8 to 1.25 v/v, preferably from 0.9 to 1.1 v/v, in particular around 1 v/v, is dried to constant weight at 50-60°C, preferably under vacuum at approximately 40°C. Karl-Fischer analysis of the resulting product shows that novel crystalline form B of fexofenadine hydrochloride hydrate has a water content ranging from about 3 to 5%, so that it can be properly defined as "approximately monohydrate".

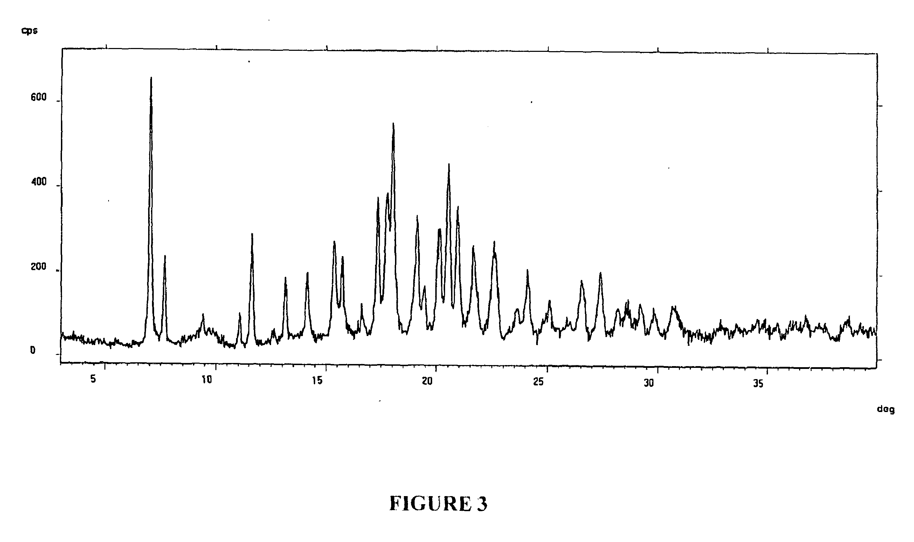

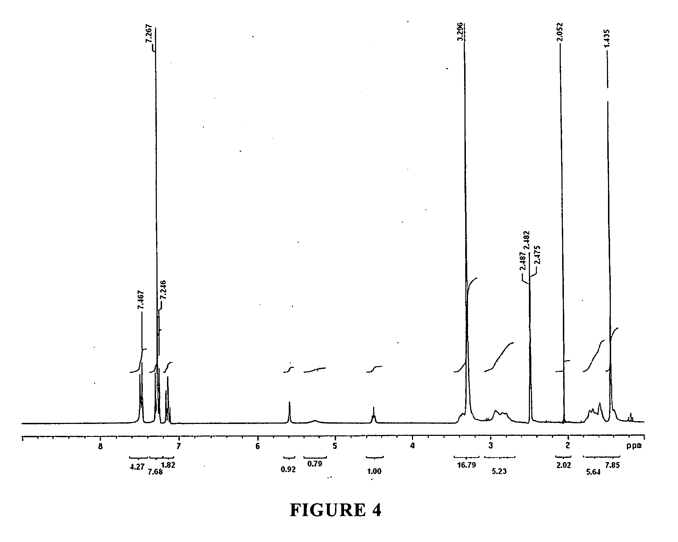

In a second aspect, the invention provides a novel crystalline form of fexofenadine hydrochloride, approximately monosolvate with acetonitrile, hereinafter referred to as form C, having an XRPD spectrum substantially as illustrated in figure 3, wherein the most intense diffraction peaks fall at 7.0; 11.6; 15.4; 17.3; 18.0 and 20.5 in 2θ; and a ¹H-NMR spectrum substantially as illustrated in figure 4. The novel form can be prepared by means of a process comprising the following steps:
- preparation of a dispersion of fexofenadine hydrochloride hydrate in acetonitrile;
- heating of the dispersion at a temperature ranging from about 70°C to the reflux temperature;
- cooling of the dispersion at a temperature lower than -5°C; and
- separation of the precipitate.

Fexofenadine hydrochloride hydrate, used as starting material, can be prepared according to known methods, for example as disclosed in WO 95/31437, or can be the novel form B which is disclosed herein.

The acetonitrile dispersion is heated for a time ranging from about 15 minutes to one hour, preferably from 30 to 45 minutes, then cooled preferably to a temperature ranging from about -15 to -10°C, for a time sufficient to obtain complete precipitation of fexofenadine hydrochloride, approximately monosolvate with acetonitrile, typically from about 15 minutes to 8 hours, preferably for about 5 hours.

According to a preferred aspect of the invention, the dispersion of fexofenadine hydrochloride hydrate in acetonitrile is obtained pouring fexofenadine hydrochloride hydrate in acetonitrile, previously heated to about 35-60°C, preferably to about 40-55°C, in particular 45°C, in a weight/volume ratio ranging from about 1/8 to 1/15, preferably of about 1/10. Fexofenadine hydrochloride hydrate is added to acetonitrile until all the fexofenadine is dissolved, keeping the temperature between about 70°C and the reflux temperature. During this phase fexofenadine hydrochloride form C starts to crystallise and precipitate. The acetonitrile dispersion is cooled to a temperature lower than -5°C, preferably to about -15/-10°C, in about four hours. The precipitate is then separated with known techniques, for example filtration or decantation of the solvent, preferably filtration. The product is then dried at a temperature of about 60°C or lower, preferably under vacuum at approximately 50°C for 10 hours. NMR analysis of the resulting product shows that novel crystalline form C of fexofenadine hydrochloride has an acetonitrile content ranging from about 6.5 to 7.5%, therefore it can be properly defined as "approximately monosolvate".

The skilled chemist will note that the conditions to obtain the dissolution of fexofenadine hydrochloride in methanol/water, or the dispersion of fexofenadine monohydrate in acetonitrile, can be modified without altering the form of the resulting polymorph. Likewise, the crystallization techniques may slightly differ from those herein described without altering the polymorphic form obtained. For example, crystallization can be promoted by addition of pre-formed crystals.

A further object of the invention is a process for the preparation of anhydrous fexofenadine hydrochloride form A, disclosed in WO 02/066429, comprising removing acetonitrile from crystals of fexofenadine hydrochloride form C, as herein defined.

The removal of acetonitrile from fexofenadine hydrochloride form C can be carried out with any suitable physical means, preferably by heating under vacuum at about 80-110°C for a time ranging from about 18 to 70 hours, more preferably at about 100°C under vacuum for about 24-30 hours.

The novel fexofenadine hydrochloride forms B and C are useful for the administration of fexofenadine in mammals in the need of said treatment. They can be administered alone or in combination or in admixture with one or more known polymorphic forms of fexofenadine hydrochloride, for example those disclosed in WO 02/066429, US 2003/0021849 or WO 95/31437. The content of each form in the mixtures depends on their physical and biological properties and will be determined by the skilled person. A variety of pharmaceutical compositions can be prepared for the administration in humans or animals, according to known techniques. The amount of fexofenadine hydrochloride in capsules, tablets, sugar-coated pills or other forms for the single administration ranges from about 30 to about 180 mg per dose unit. Therefore, the invention also provides a pharmaceutical composition comprising, as active ingredient, fexofenadine hydrochloride form B and/or C, or mixtures of at least one of them with one or more known polymorphic forms of fexofenadine hydrochloride in admixture with a suitable carrier and/or excipient. Said pharmaceutical composition can also optionally contain a therapeutically effective amount of pseudoephedrine.

The following examples illustrate the invention.

### Example 1: Preparation of fexofenadine hydrochloride form B

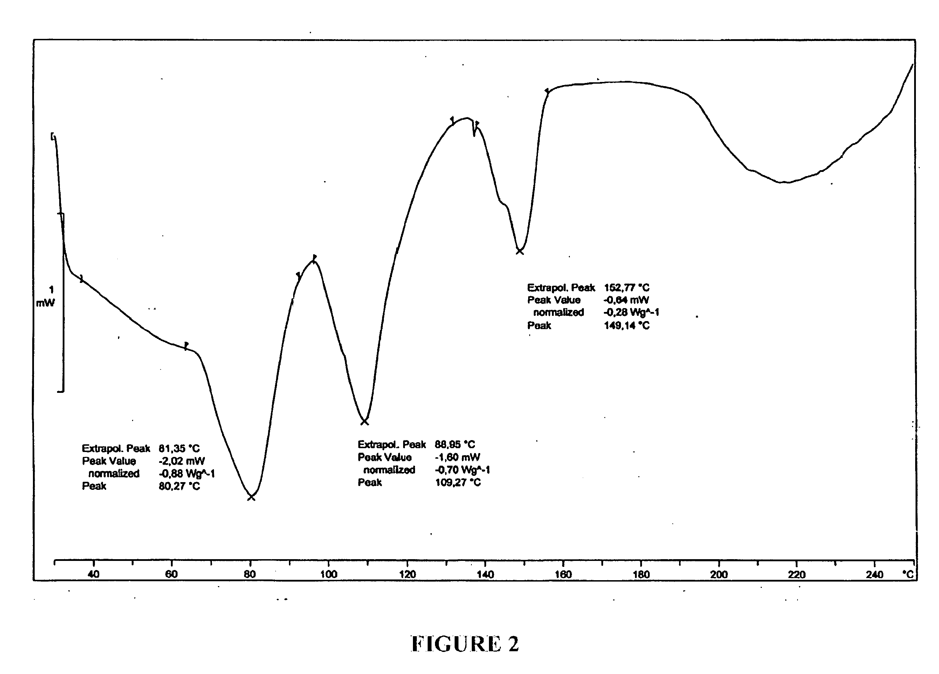

278 g of 4-[4-[4-(hydroxydiphenylmethyl)-1-piperidinyl]-1-oxobutyl]-α,α-dimethylbenzeneacetic acid, 355 ml of water, 765 ml of methanol and 22.6 g of sodium hydroxide pellets are loaded in a 3 litres four-neck flask. The resulting mixture is heated to 40°C, then a solution of sodium borohydride (9.8 g) in 47 ml of water is added. When the reduction reaction is complete, the mixture is added with 40 ml of acetone and the pH is adjusted to a value ranging from 2.5 to 3 with 36% w/w hydrochloric acid. Thereafter, 140 ml of methanol and 450 ml of water are added, keeping the temperature at 40°C. The resulting mixture is slowly cooled to -15°C and the resulting precipitate is filtered and washed with a 1/1 water/methanol mixture. The resulting product is dried under vacuum at 40°C to constant weight. 265 g of fexofenadine hydrochloride are obtained with a 4.1 % Karl-Fischer water content (therefore it can be referred to as monohydrate) and substantially characterised by the XRPD and DSC spectra reported in Figures 1 and 2.

### Example 2: Preparation of fexofenadine hydrochloride form C

2650 ml of acetonitrile are loaded in a four-necked 3 litres flask, then heated to 45°C. 265 g of fexofenadine hydrochloride hydrate is added under stirring and the suspension is refluxed at (80-82°C) for about one hour. The suspension is cooled to about -15/-10°C in about 4 hours. The precipitated solid is filtered and washed with acetonitrile (2 x 80 ml) to obtain 285 g of wet product which, after drying under vacuum at 50°C for 10 hours, yields 262 g of fexofenadine hydrochloride monosolvate with acetonitrile, having a XRPD spectrum and a ¹H-NMR spectrum substantially as illustrated in Figures 3 and 4 respectively and a 0.25% Karl-Fischer water content.

### Example 3: Preparation of fexofenadine hydrochloride form A

262 g of fexofenadine hydrochloride form C, obtained as described in Example 2, are ground and dried under vacuum at 100°C for 24 hours. 244 g of fexofenadine hydrochloride anhydrous form A are obtained, having a melting point between 153 and 156°C and a XRPD spectrum having substantially the same characteristics as those reported in table 1 of WO 02/066429.

## Claims

1. Crystalline form of fexofenadine hydrochloride, approximately monosolvate with acetonitrile, having an XRPD spectrum substantially as that reported in Figure 3.

2. The crystalline form of fexofenadine hydrochloride according to claim 1, having an XRPD spectrum wherein the most intense diffraction peaks fall at 7.0; 11.6; 15.4; 17.3; 18.0 and 20.5 in 2θ.

3. The crystalline form of fexofenadine hydrochloride according to claim 1 or 2, having a ¹H-NMR spectrum substantially as that reported in Figure 4.

4. A process for the preparation of fexofenadine hydrochloride, approximately monosolvate with acetonitrile, as defined in claim 1, comprising the following steps:
- preparation of a dispersion of fexofenadine hydrochloride hydrate in acetonitrile;
- heating of the acetonitrile dispersion at a temperature ranging from about 70°C to the reflux temperature;
- cooling of the acetonitrile dispersion to a temperature lower than -5°C; and
- separation of the precipitate.

5. The process according to claim 4, wherein the dispersion of fexofenadine hydrochloride hydrate in acetonitrile is obtained pouring fexofenadine hydrochloride hydrate in acetonitrile, previously heated to about 35-60°C, in a weight/volume ratio ranging from about 1/8 to 1/15.

6. The process according to claim 4 or 5, wherein the acetonitrile dispersion is cooled to a temperature of about -15/-10°C.

7. A process for the preparation of anhydrous fexofenadine hydrochloride form A, as designated in WO 02/066429, comprising the removal of acetonitrile from crystals of approximately monosolvate fexofenadine hydrochloride with acetonitrile, as defined in claim 1.

8. The process according to claim 7, wherein acetonitrile is removed by heating under vacuum at about 80-110°C for a time ranging from about 18 to 70 hours.

9. Pharmaceutical composition comprising, as active ingredient, fexofenadine hydrochloride approximately monosolvate with acetonitrile, as defined in claim 1, or a mixture thereof with one or more known polymorphic forms of fexofenadine hydrochloride, in admixture with a carrier and/or excipient.

10. The pharmaceutical composition according to claim 8, further containing pseudoephedrine, as active ingredient.
